# EUROPEAN PATENT APPLICATION

(11) **EP 3 632 408 A1**
(43) Date of publication of application: **08.04.2020**
(21) Application number: 19201228.4
(22) Date of filing: 03.10.2019
(51) Int. Cl.: A61K 8/9761, A61K 8/9783, C08L 97/02

(54) **WOOD PARTICLES MICROBEADS ENRICHED WITH BERRY (MAINLY: 1- BILBERRY: VACCINIUM MYRTILLUS/2- LINGONBERRY: VACCINIUM VITIS-IDAEA / 3- AMERICAN BLUEBERRY: VACCINIUM CYANOCOCCUS) EXTRACT**

(30) Priority: 04.10.2018 SE 1851195
(71) Applicant: Seido, Jim, 962 31 Jokkmokk (SE)
(72) Inventor: Seido, Jim, 962 31 Jokkmokk (SE)
(74) Representative: Zacco Denmark A/S

(57) **Abstract**

A method for producing wood particles microbeads enriched in berry extract, wood particles microbeads, and uses thereof in industrial, home and personal care products.

## Description

### TECHNICAL FIELD

The application is in the field of microbeads from natural sources, such as wood particles and their uses, in particular the use of such microbeads in the home and personal care products and relevant industrial applications.

### BACKGROUND

Plastic microbeads are manufactured solid plastic particles of less than one millimetre in their largest dimension. They are most frequently made of polyethylene but can be of other petrochemical plastics such as polypropylene and polystyrene. Classic microbeads are commonly used in exfoliating personal care products, toothpastes and detergents. Sphericity and particle size uniformity create a ball-bearing effect in creams and lotions, resulting in a silky texture and spreadability. Smoothness and roundness can provide lubrication.

Despite the above-mentioned advantages plastic microbeads have a severe environmental effects. Since the plastic particles are so small they are easily washed down the drain and thereafter pass the sewage treatment plants and finally end up in rivers and channels. This results in pollution of water with plastic particles. A large number of fish and animals mistake plastic microbeads for their food source and thereby consume toxic plastic.

From 2013 many countries started to impose a ban on plastic microbeads in the products. In 2018 UK ban came into effect. Plastic microbeads can no longer be used in cosmetics and personal care products in the UK. The Canadian ban on microbeads also came into effect on January 2018, when Ireland is expected to ban microbeads by the end of 2018. Sweden also banned microbeads in cosmetic products "rinsed or spotted and which contain plastic particles whit a cleaning scrubbing or polishing function" since beginning of July 2018.

In view of the above ban there is a need for developing an alternative source of the micro particles with the same effect as plastic microbeads, however, less harmful for the environment. There is also a need and a high customer demand for products, naturally enriched with vitamins and anti-oxidants, which are produced in the environment-friendly way.

WO02/22172 discloses a fibrous cellulose excipient suitable for use as a binder, filler, and/or disintegrate in the development of solid dosage forms and as drug carrier in the preparation of topical formulations. The cellulose excipient is formed by soaking a source of cellulose in an aqueous alkali metal hydroxide solution. The cellulose is then regenerated, washed, and optionally hydrolysed with a dilute mineral acid. The cellulose excipient is also useful as an aqueous dispersion in topical formulations and in the manufacture of cellulose beads. However, this method involves chemical treatments and modification of the cellulose source, such as wood particles. This is not environmentally friendly and, moreover, increases productions costs. Moreover, the final product described is a dispersion, and is not homogeneous.

In view of above there is a further need for a new ecological and environmentally friendly source of natural microbeads and the method for production thereof, wherein the method does not involve any chemical treatment of the raw materials.

The problem is solved in accordance with the invention by providing the wood particles microbeads, enriched with berry extracts and new uses thereof, in particular in home and personal care products, and for industrial use as an abrasive agent.

### SUMMARY OF THE INVENTION

In a first aspect of the present invention it is disclosed a method for producing wood particles microbeads enriched in berry extract, the method comprising providing wood particles and berries, said wood particles having a length in its largest dimension of 3 mm or less, mixing wood particles with berries in a ratio between 1:5 to 5:1 of wood to berries (w/w), heating said mixture of wood particles and barriers at between 40°C to 75°C for a time sufficient for wood particles to absorb berry extract, thereby forming a first mixture of wood particles enriched with the berry extract, wherein water content of said wood particles enriched with berry extract in the first mixture is 75% or less, subjecting said first mixture to size reduction procedure, thereby obtaining a second mixture of wood particles microbeads enriched with berry extract, wherein said microbeads have a size of 1 millimetre or less in their largest dimension and are uniformed in size.

In another aspect it is disclosed the method, wherein said second mixture is subjected to drying at a temperature of between 40°C to 75°C for a time sufficient to decrease water content in said second mixture to 70% or less, preferably 60% or less, preferably 50% or less, more preferably 40% or less, more preferably 30% or less, more preferably 20% or less, more preferably 10 % or less.

In yet another aspect it is disclosed the method, wherein said second mixture is optionally dried to a water content of 50% or less, 30% or less, 15% or less, 5% or less.

In yet another aspect it is disclosed the method, wherein said second mixture is a powder.

In yet another aspect it is disclosed the method, wherein said second mixture is further diluted with diluent to a water content sufficient to be subjected to a size reduction procedure.

In yet another aspect it is disclosed the method, wherein said wood particles are selected from hard wood or soft wood.

In yet another aspect it is disclosed the method, wherein the soft wood is selected from cedar, juniper, pine redwood, spruce.

In yet another aspect it is disclosed the method, wherein the hard wood is selected from alder, balsa, beech, birch, hickory, mahogany, maple, oak, teak, and walnut.

In yet another aspect it is disclosed the method, wherein the berries are selected from bilberries, lingberries, blueberries, raspberries, red currants or black currants.

In yet another aspect it is disclosed the method wherein the ratio between berries and wood particles is at between 1 parts of berries to 5 parts of wood particles (w/w) to 5 parts of berries to 1 part of wood particles (w/w).

In yet another aspect it is disclosed wood particles microbeads obtained by the method of any of the preceding aspects.

In yet another aspect it is disclosed wood particles microbeads enriched in berry extract, said wood particles of 1 milometer in the particle largest dimension or less and are uniform in size, wherein water content of wood particles microbeads is 75% or less.

In yet another aspect it is disclosed a wood particles microbeads according to the previous aspect, wherein said wood particles are selected from hard wood or soft wood.

In yet another aspect it is disclosed wood particles microbeads, wherein the soft wood is selected from cedar, juniper, pine redwood, spruce.

In yet another aspect it is disclosed wood particle microbeads according to some previous aspects, wherein the hard wood is selected from alder, birch, balsa, beech, hickory, mahogany, maple, oak, teak, and walnut.

In yet another aspect it is disclosed wood particle microbeads according to the previous aspects, wherein the berries are selected from bilberries, lingberries, blueberries, raspberries, red currants or black currants.

In yet another aspect it is disclosed a use of wood particles microbeads according to the previous aspects as an exfoliating and/or abrasive agent in industrial, home and personal care products.

In yet another aspect it is disclosed a use of wood particles microbeads as defined in the previous aspect, wherein industrial products are selected from engine grease and oil remover, paint remover, industrial hand wash.

In yet another aspect it is disclosed a use of wood particles microbeads according to the previous aspects, wherein said home and personal care products are selected from detergents, soaps and cosmetic products.

### DETAILED DESCRIPTION

### DEFINITIONS

### Wooden particles

Wooden particles are typically originated as a by-product from wood working operation such as sawing, milling and sanding. These operations both shatter lignified wood cells and break out whole cells and groups of cells. Shattering of wood cells creates dust, while breaking out of whole groups of wood cells creates chips. The more cell-shattering that occurs, the finer the dust particles that are produced. For example, sawing and milling are mixed cell shattering and chip forming processes, whereas sanding is almost exclusively cell shattering is generated in large quantities in wood working industry and is often disposed as a waste. However, in the context of the present invention saw dust obtained as a result of cell shattering is used directly in accordance with the present invention. While chips of wood can be chopped into the smaller size suitable for use in accordance with the method of the resent invention and thus are also encompassed by the definition of the present invention.

### Berries

Berries suitable in accordance with the invention are defined in a common sense and not in a purely scientific usage.

The scientific term berries is defined as a fruit produced from the ovary of a single flower in which the outer layer of the ovary wall develops into an edible fleshy portion (pericarp). The definition includes many fruits that are not commonly known as berries such as tomatoes, cucumbers and eggplants, which is not the intention of the present invention.

In accordance with the present invention the berries are defined by a common usage of the term and are typically bilberries, lingonberries, blueberries, raspberries, black and red currants etc.

### Water content

Water content or moisture content is the quantity of water contained in a material and is expressed as a ratio, which can range from 0 (completely dry) to the value of the materials' porosity at saturation. Sometimes water content may be defined as moister content.

### Industrial care products

Industrial care product typically include heavy duty hands cleaner, which remove grease, dirt and industrial oil, paints.

### Home and personal care products

Daily chemical products are but not limited to detergents, soaps, shampoos, body scrubs, cosmetic.

### Cosmetics

Cosmetics are substances or products used to enhance or alter the appearance of the face or fragrance and texture of the body. Many cosmetics are designed for use of applying to the face, hair, and body. They are generally mixtures of chemical compounds; some being derived from natural sources (such as coconut oil), and some being synthetics or artificial. The common non-liming examples of cosmetics include lipstick, mascara, eye shadow, foundation, skin cleansers and body lotions, shampoo and conditioner, hairstyling products (gel, hair spray, etc.).

### Detergents

Detergent in the context of the present invention means a surfactant or a mixture of surfactants with cleaning properties in dilute solutions. These substances are typically alkyl benzenesulfonates, a family of compounds that are similar to soap but typically are more soluble in hard water, because the polar sulfonate (of detergents) is less likely than the polar carboxylate (of soap) to bind to calcium and other ions found in hard water. In the context of present inventions soaps and other washing solutions are also a part of the invention.

### Size reduction system

Raw materials such as wood chips often are present in sizes that are too large to be used directly and therefore, they must be reduced in size. Depending on whether material is a solid or a slurry, the operation of size reduction may be divided into two major categories. In case of solids the operations are called grinding and cutting, while in the case of slurry, the process is defined as pulverisation. Therefore, within the scope of present invention, both grinding and pulverization may be equally employed depending on the water content of the original wood particles enriched with berry extract.

### Figure legends

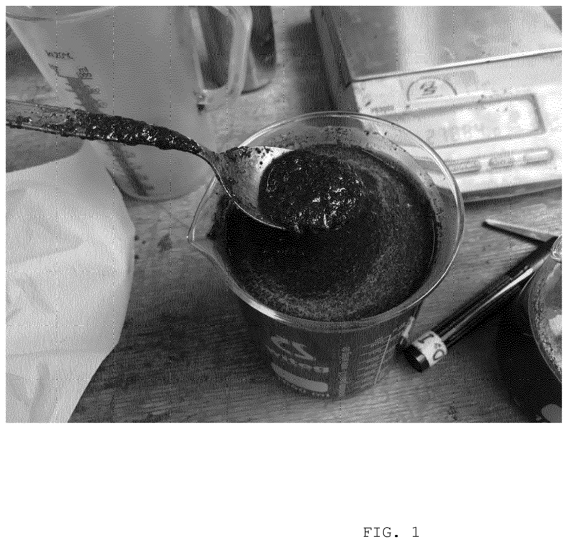

Figure 1 is a picture of wood particles enriched with berry extract prior to subjection to a size reduction procedure. In this case pulverisation procedure, since the moisture content of the wood particles enriched in berry extract is 75%.
Figure 2 is a picture of a wood particles microbeads of a size of at most 1 millimetre in their largest dimension obtained on the Micros device in accordance with the method of the invention.
Figure 3 is a graph of a particle size distribution after processing in Micros device dependent on a processing time in the first test T1 and a second test T2.

### DETAILED DESCRIPTION

In accordance with the method for producing wood particles microbeads enriched in berry extract, the method comprising providing wood particles and berries, said wood particles having a length in its largest dimension of 3 mm or less, mixing wood particles with berries in a ratio between 1:5 to 5:1 of wood to berries (w/w), heating said mixture of wood particles and barriers at between 40°C to 75°C for a time sufficient for wood particles to absorb berry extract, thereby forming a first mixture of wood particles enriched with the berry extract, wherein water content of said wood particles enriched with berry extract in the first mixture is 75% or less, subjecting said first mixture to size reduction procedure, thereby obtaining a second mixture of wood particles microbeads enriched with berry extract, wherein said microbeads have a size of 1 millimetre or less in the particle's largest dimension and are uniformed in size.

The wood particle microbeads enriched in berry extract produced by the method and intended for use in accordance with some aspects of the invention are berry bio-powder microbeads with cellulose particles from wood of trees and/or fruit cores and/or nut sheels.

While traditional plastic microbeads are perfectly round in shape, it shall be understood that the wood microbeads produced in accordance with the method as defined herein, may slightly deviate from the ideally round shape. Therefore, the measurement are typically done in any dimension, however in any case none of the measurements shall exceed 1 mm.

In the solid wood products industry, water content is typically defined on a dry basis. This means that the moisture content (or water content) is defined as WC is equal to Weight of water/ Weight of wood times 100% (Formula 1). The weight of the wood does not include any water. It is the weight of the piece or particle mixture after oven-dry, wherein all water has been removed. The weight of the water is the difference in the weight of the piece before and after drying. Therefore, when the test is done the following formula is used to calculate water content. Water content is equal to (Initial weight - Oven-dry weight)/Oven - dry weight x 100% (Formula 2). Formulae one and two are the same, Formula 1 is conceptual, formulae 2 is how the water content is actually calculated in practice.

Initially wood particles, wherein each individual particle has the length of largest dimension of 3 mm or less are provided. The particles are typically not homogeneous in size. Smaller wood particles of 3 mm length in their largest dimension or less are preferred since they can be directly used in the method in accordance with the present invention and be subjected to the size reduction procedure after enrichment with the berries extract. The inventors observed that the particles of this size absorb berry extract in the sufficient amounts.

However, the particles of a size larger than 3 mm in the particle's largest dimension can also be used according to the main principle of the present invention. In such case the wooden particles are crushed or broken into the particles of a size suitable for the use in a method. The resulting particles have to be sufficiently small but do not have to be homogeneous in size.

Therefore, wood chips are also suitable as a starting material. Moreover, larger leftovers of the wood industry can be chopped into the pieces of a desired size. The wood particles and saw dust can be purchased from eco wood industry leftovers, or even if necessary larger wooden pieces may be bought and crash into the pieces of a desired size. This is yet another advantage on the present invention allowing utilization of wood industry leftovers in an ecological and cost efficient way.

Contrary to cellulose microbeads, described in the prior art; wood particles are ready available, typically as a by-product in wood industry. No further purification or extraction is needed and they can be used immediately. The only preparation of wood particles in accordance with the invention is crushing them into the desired size and enriching with berry extract, which means that there is a pure mechanical treatment of the material already available. No additional treatment with chemicals is necessary.

Typically classic plastic microbeads have the advantage of round and smooth shape and therefore are highly suitable for use in cosmetics, facial products and soaps.

Surprisingly, the inventor has found out that mechanical crashing of the wood particles results in the particles sufficiently smooth and small to be suitable as a substitute for plastic microbeads. Also as it is apparent from the examples below that wood particles enriched with berry extract can be crushed into the desired size, comparable with the size of microbeads, thus providing a method of producing microbeads having natural origin, i.e. wood microbeads.

Berries used in the present invention may be fresh berries, frozen berries or berry juice concentrate. Berries can be single berry type or a mixture of different types of berries. Berries according to the invention can be selected dependent on the intended use of the wood particles microbeads. For example, bright coloured berries like bilberries might be less desirable for use in detergents. However, they are more suitable for use in soaps and other cosmetics.

The type of berries used is not limiting in the context of the present invention and any other berries may be equally used for the same purpose.

It is the inventor's idea that wood chops, which are naturally porous can be crushed into the small particles and at the same absorb nourishing elements such as antioxidants and vitamins from the berries, can be used to substitute plastic microbeads in various products.

The wood particles and the berries are mixed in any container suitable for the purpose. There is no limit on how to mix the proportions, and it is largely depend on how much the wood can absorb, which in turn determined by the type of the wood used. Typically, hard porous wood can absorb more than the soft non porous wood. Another limitation is the intended use of the mixture and may be defined by the end-use product. For instance if the mixture is intended to be used as a scrub, the wood particles content in the mixture can be higher, such as up to 70-80%. If the mixture is intended to be used as a face cleanser, for example, the amount of wood particles can be as low as 16%. In accordance with the invention the proportion can be between 1 : 5 to 5:1 wood/berries.

The ratio between volume of wood particles to berries can be between 2:4; 3:3; 4:2; 2:4; 5:1.

It has been observed by the inventor that under continuous heating at the temperature between 40°C to 75°C water slowly evaporate from the mixture leaving the wooden particles enriched with berry extract. The temperature may be between 45°C to 70°C, 50°C and 65°C, between 55°C to 60°C.

At the same time antioxidants and oils comprised in the berries will be extracted from the crushed berries and absorbed by the wood particles from the berries. The berry extract absorbed would not be evaporated from wood particles under these conditions.

It has been observed by the inventor that under continuous heating at the above referenced temperatures wood absorbs berry juice and at the same time vitamins and natural colorants present in berries are not destroyed by high temperatures or mechanical treatment. At the same time antioxidants and oils comprised in the berries will be extracted from the crushed berries and absorbed by wood particles.

To complete the process, the mixture is further heated until the majority of water evaporates. The temperatures of maximum 75°C are sufficient for slow evaporation of water and at the same time preserve the vitamins. Temperature above 80°C are not suitable since elevated temperature may destroy the vitamins.

The continuous heating is maintained until the sufficient amount of water evaporates from the final product. The sufficient amount is determined by the intended use of a product and is not limiting on the scope of present invention. In some circumstances in it might be sufficient that water is evaporated until the moisture content of wood particles enriched with the berry extract is about 70%. 70% water content is particularly suitable for graining the particles to the smaller size by device in Example 1. However, said wood particles enriched with berry extract may be dried to the water content of less than 70%, less than 60%, less than 50%, less than 40%, less than 30%, less than 20%, less than 10% or 0% and be a powder, and further grained by any device suitable for reducing the size (for example, graining dry materials) and in particular, powdered materials.

Alternatively, the dried material may be diluted by water to the desired moisture content (or water content) and subjected to further graining procedure in Micros device, as exemplified below.

Thus, water content can be 70% or less, 60% or less, 50% or less, 40% or less, 30% or less, 20% or less, 10% or less. Water content of the mixture can be as low as 3% or 2%. When water content is 3% or 2% or less the composition obtained by the method is essentially a powder.

Water content can be defined by for example, loss-on-drying method, using CSC digital moisture balance device (http://www.cscscientific.com/moisture/loss-on-drying/digital-moisture-balance) .

Alternatively, water content may be defined by Karl Fischer method of moisture detection on CSC Karl Fischer Oven/Evaporator System (http://www.cscscientific.com/karl-fischer-oven-evaporator) . Further, the mixture is subjected to a size reduction procedure by, for example, graining, crushing or pulverizing. Wood particles enriched with the berry extract are grained to the size of at most 1 millimetre in the largest dimension to the size as small as 10 micrometres. The size is defined by a final use of the product and is not limiting on the scope of present invention insofar the particles are of less than 1 millimetre in its largest dimension.

In accordance with the invention said wood particles microbeads enriched with berry extract mixture is subjected to drying at a temperature of between 40°C to 75°C for a time sufficient to decrease water content in the said mixture to 70% or less, preferably 60% or less, preferably 50% or less, more preferably 40% or less, more preferably 30% or less, more preferably 20% or less,

Further in accordance with the method, said second mixture is optionally dried to a water content of 50% or less, 30% or less, 15% or less, 5% or less. The second mixture may be left to comprise about 70% water in said mixture or dried further to a desired water content.

Further said second mixture can have a very low water content, such as 5% or less, such as 3% or less, such as 2% or less and be essentially a powder.

Furthermore, in a method in accordance with the present invention said second mixture may be diluted with water to a water content sufficient for subjecting to subsequent size reduction procedure. Amount of water added to the second mixture depends on size reduction protocol and a type of the device used.

It is further disclosed that in accordance with the method wood particles are selected from hard wood or soft wood.

Hard wood used in accordance with the invention is more porous and has an advantage of being capable of easy absorption of the berry extract. Typical example of the hard wood is teak wood, birch, alder, beech, mahogany, maple, oak, teak and walnut.

Soft wood in accordance with the invention may be but not limited to cedar, juniper, pine redwood, spruce.

Which type of wood is used for a particular application may be defined by, for example, the type of wood available due to the landscape or the type of wood industry present in the area.

It shall be understood that the type of wood is not limiting on the concept of the present invention.

It is further disclosed a method for producing wood particles microbeads enriched in berry extract, wherein the berries are selected from bilberries, lingberries, blueberries, raspberries, red currants or black currants.

In accordance with one embodiment bilberries are most preferred. Bilberries are naturally more acidic in nature, and an additional benefit of using bilberries is that the acidity brings down the pH in the detergents, which has typical a basic pH. Thereby, the pH of the detergents, such as soaps produced in accordance with this method is close to a natural skin pH. The detergents, such as soaps obtained by the claimed method minimise negative effect on a skin.

Bilberry (*Vaccinium myrtillus)* with the wood particles in accordance to one preferable aspect of the invention are composed as a wine colour powder, that has PH less than 7 which works not just as a peeling agent to scrub the skin, but also helping the cosmetic manufacturers to control the level of the PH under 7 without adding other chemical ingredients. This method helps produces to go more eco friendly, provide products without chemicals and reduce the cost. Another advantage is to provide a healthier and natural product for direct contact with the skin. A further advantage to the manufacturers and environment is that the Bilberry bio-powder has natural red colour and that the colour could be adjusted by the by the manufacture, such as cosmetic producer. The manufacture, such as by changing the PH number where the color range will be pass the colours from light reddish then brown to green then purple and dark blue.

Most of the bio powder now in the market are based on the cores of olive, apricots and majority of them have the wooden colour which will make the cosmetics producers add colouring materials for a better looking. The bilberry bio-powder has naturally the colouring effect without adding any chemical colours. This saves costs and reduces the chemical ingredients in the products.

When the bilberry bio-powder is added to the cream as a peeling ingredient, it will acquire the natural pink/reddish colour

In particular, bilberries colour is pH dependent; the colour is red at pH < 2, changing to blue as pH increases and finally becoming colourless at high pH.

In a preferred aspect of the method according to the invention the pH of the mixture is adjusted such that the colour of the mixture is controlled. The pH may be 1,9 such that the colour of the mixture is red, or pH may between 2 and 4, such as between 3 and 5, such as 4, preferably between 4 and 6, such 5, preferably between 5 and 7 preferably 6, such as between 7 and 9, preferably 8, such as between 10 and 12, preferably 11, such as between 11 and 13, preferably 12.

By controlling means that the pH is adjusted by adding a base or an acid to the mixture, such that the pH vale corresponds to the desired colour. Thereby desired colouring of the mixture comprising wood particles and bilberries is achieved.

The berries above a preferred but not limiting on the scope of the invention. Other berries are also suitable. Also the berries do not have to be the fresh berries, they may be frozen berries or berry juice.

The preferable berries are selected from Bilberry (*Vaccinium myrtillus),* lingonberry (*Vaccinium vitis-idaea) and* american blueberry (*Vaccinium cyanococcus)*

The method according to any of the preceding claims, wherein the wood particles and berries are mixed in a ration, sufficient for providing exfoliating effect and reduction of oxidative stress in the skin as well as improved skin appearance.

Different indications might require a different degree of exfoliating effect and anti-oxidative activity of the berries can vary depending on the type of the berries used, which in turn defines the concentration of berries in the composition.

The method according to any of the preceding claims, wherein the wood particles and berries are mixed in a ration, sufficient for providing exfoliating effect and reduction of oxidative stress in the skin.

Different indications might require a different degree of exfoliating effect and anti-oxidative activity of the berries can vary depending on the type of the berries used, which in turn defines the concentration of berries in the composition.

When the ration of between 80% of wood particles and 20% crushed berries, the composition would have predominantly exfoliating effect with a mild nourishing and anti-oxidative effect, as well as improved skin appearance. If more anti-oxidative effect is desired from the compositions the ratio can be changes up to 20% wood particles and 80% crashed berries.

The best combination of the exfoliated and anti-oxidative and nourishing, improved appearance effects, is achieved when the ratio of wood particles and crashed berries is 1:1.

However, it is not limiting on the context of the present invention the exact proportion of the two ingredients.

The inventive concept is the unexpected combination of the naturally developed microbeads, enriched in the antioxidants and other natural ingredients.

Yet another advantage of the invention that due to the different colours of the berries used the final product can have different colour.

In particular, typically plastic microbeads are essentially colourless. Addition of such microbeads to the final products, in particular detergents such as soaps, creates a visual impression of white spots. This is in particular visible, when the product itself has a bright colour. Microbeads obtained by the method described in this disclosure, are coloured due to the natural colours presented in the berries, such as bilberries.

This allows not only the aesthetic advantage but also permits colour coding of various types of the product. The colouring agent used in natural and absorbed in the pores of the wooden particles, thereby reducing a need for any type of chemical components in the cosmetic product. Thus resulting in natural paint, reducing use of the chemicals.

Furthermore, the colour can be controlled by additional control of pH of the final product. Anthocyanins are pH indicators: they change color depending on if they are in an acidic, neutral, or basic (alkaline) environment.

As a non-limiting example of this embodiment of the invention, the soap can be dark purple due to blueberries used in combination with wooden particles and facial cream can be, for example red due to berries used in accordance with the present invention. This can ease use of the products by a consumer.

The method described above allows for obtaining wood particles microbeads enriched with the berry extract.

It is further disclosed wood particles microbeads enriched in berry extract, said wood particles having the size of 1 milometer in the largest dimension or less and are uniform in size and a water content of wood particles microbeads is 75% or less. These microbeads are entirely naturel and thus do not have any negative impact on the environment, contrary to plastic microbeads. Furthermore, these microbeads use leftovers of wood industry. Dues to the berry extract absorbed by wooden particles the microbeads are enriched with vitamins, minerals and antioxidants. The microbeads are also naturally coloured that decrease use of synthetic colouring ingredients in the final cosmetic or industrial product.

It is possible to control the size of said wood particles microbeads enriched with berry extract, and they can be as small as 9 µm, as exemplified below.

Further it is disclosed herein wood particles microbeads, wherein said wood particles are selected from hard wood or soft wood. Non limiting examples of soft wood are cedar, juniper, pine redwood, spruce. Non limiting examples of hard wood are alder, balsa, beech, birch, hickory, mahogany, maple, oak, teak, and walnut.

It can be contemplated that wood particles microbeads are produced from fruit cores or nut shells.

Non- limiting examples of berries used for wood particles microbeads are bilberries, lingberries, blueberries, raspberries, red currants or black currants.

Wood particles microbeads enriched with berry extracts may be used as exfoliating and /or abrasive agent in industrial, home and personal care products.

Non limiting examples of industrial care products are engine grease and oil remover, paint remover, industrial hand wash. Non limiting examples of home and personal care products are detergents, soaps, cosmetic products.

### EMBODIMENTS:

1. A method for producing wood particles microbeads enriched in berry extract, the method comprising
   - providing wood particles and berries, said wood particles having a length in its largest dimension of 3 mm or less,
   - mixing wood particles with berries in a ratio between 1:5 to 5:1 of wood to berries (w/w),
   - heating said mixture of wood particles and barriers at between 40°C to 75°C for a time sufficient for wood particles to absorb berry extract, thereby forming a first mixture of wood particles enriched with the berry extract, wherein water content of said wood particles enriched with berry extract in the first mixture is 75% or less,
   - subjecting said first mixture to size reduction procedure, thereby obtaining a second mixture of wood particles microbeads enriched with berry extract, wherein said microbeads have a size of 1 millimetre or less in their largest dimension and are uniformed in size.
2. The method according to embodiment 1, wherein said second mixture is subjected to drying at a temperature of between 40°C to 75°C for a time sufficient to decrease water content in said second mixture to 70% or less, preferably 60% or less, preferably 50% or less, more preferably 40% or less, more preferably 30% or less, more preferably 20% or less, more preferably 10 % or less.
3. The method according to any of embodiments 1 to 3, wherein said second mixture is optionally dried to a water content of 50% or less, 30% or less, 15% or less, 5% or less.
4. The method according to embodiment 4, wherein said second mixture is a powder.
5. The method according to any of embodiments 3 or 4, wherein said second mixture is further diluted with diluent to a water content sufficient for a subsequent use.
6. The method according to any of embodiments 1 to 5, wherein said wood particles are selected from hard wood or soft wood.
7. The method according to emodiment 6, wherein the soft wood is selected from cedar, juniper, pine redwood, spruce.
8. The method according to embodiment 6, wherein the hard wood is selected from alder, balsa, beech, birch, hickory, mahogany, maple, oak, teak, and walnut.
9. The method according to any of the preceding embodiments, wherein the berries are selected from bilberries, lingberries, blueberries, raspberries, red currants or black currants.
10. The method according to any of the preceding embodiments, wherein the ratio between berries and wood particles is at between 1 part of berries to 5 parts of wood particles (w/w) to 1 part of berries to 1 part of wood particles (w/w).
11. Wood particles microbeads obtained by the method of any of embodiments 1 to 10.
12. Wood particles microbeads enriched in berry extract, said wood particles of 1 milometer in the largest dimension or less and are uniform in size and a water content of wood particles microbeads is 75% or less.
13. Wood particles microbeads according to embodiment 12, wherein said wood particles are selected from hard wood or soft wood.
14. Wood particles microbeads according to embodiment 13, wherein the soft wood is selected from cedar, juniper, pine redwood, spruce.
15. Wood particle microbeads according to embodiment 13, wherein the hard wood is selected from alder, balsa, beech, birch, hickory, mahogany, maple, oak, teak, and walnut.
16. Wood particle microbeads according to any of embodiments 13 to 15, wherein the berries are selected from bilberries, lingberries, blueberries, raspberries, red currants or black currants
17. Use of wood particles microbeads as defined in embodiments 11- 16 as an exfoliating and/or abrasive agent in industrial, home and personal care products.
18. Use of wood particles microbeads as defined in embodiment 16, wherein industrial products are selected from engine grease and oil remover, paint remover, industrial hand wash.
19. Use of wood particles microbeads according to embodiment 18, wherein said home and personal care products are selected from detergents, soaps, cosmetic products.

### Example 1.

Berries (bilberries) with wood chips were used as raw material. The goal of the experiment was to pulverize the particles as fine as possible. The method used in this non limiting example was wet milling.

The device used in this non-limiting example is MICROS (MIC-2), material of grinding parts: ZrO₂, effective volume: 1.71.

Further equipment was particle size analysis, it was performed on Sympatec Particle size analyser, and the wet content was measured on infrared moisture meter 105°C 5g, automatic.

Two independent experiments were performed and the results can be summarised as following.

**Table 1. Parameters of the experiment**

| Test No. | | T-1 | T-2 |
|---|---|---|---|
| Material amount | g | 1100 | 1000 |
| Moisture content | %WB | 75 | 75 |
| Rotation speed | min⁻¹ | 1200 | 800 |
| Processing time | min | 25 | 40 |
| Final particle | µm | 9.6 | 9.9 |
| Material | °C | 72.5 | 73.0 |

As seen from Table 1, the initial material for this particular pulverization method needed to be diluted to a moisture content of around 75%WB to form a slurry that can be processed with the MICROS. The final particle size that can be reached is around [d₅₀]=10µm. With 1200rpm it took 20minutes to reach [d₅₀]=10µm and with 800rpm it took 40 minutes. The temperature increase was slower in the second test due to the lower rotation speed, but after 40 minutes the material temperature reached 73°C while the material during the first test reached 72.5°C after 20minutes. The amount of material processed in the equipment was reduced from 1100g in test 1 (Table 2a) to 1000g in test 2, because some material was pushed out of the equipment. With 1000g there was no more material being pushed out.

The processing of the material in the MICROS is possible and there were no problems during the operation.

### Example 2.

In the first experiment a berry and wood chips powder has been mixed with water was mixed 1:3 (w/w). 1100 g were fed into the Micros (MIC-2) device, having material of grinding part ZrO₂. Particles were grained for 5, 10, 15, 20 and 25 minutes as seen in Table 1a at a constant rotation speed 1200 min⁻¹. The temperature varied between 69°C and 75°C degrees.

It is apparent from the results that size of the particles was decreased the longer the process time was.

At already 5 minutes of pulverization, the particles were of the size of plastic microbeads and could be as small as 9.63 µm after 25 minutes.

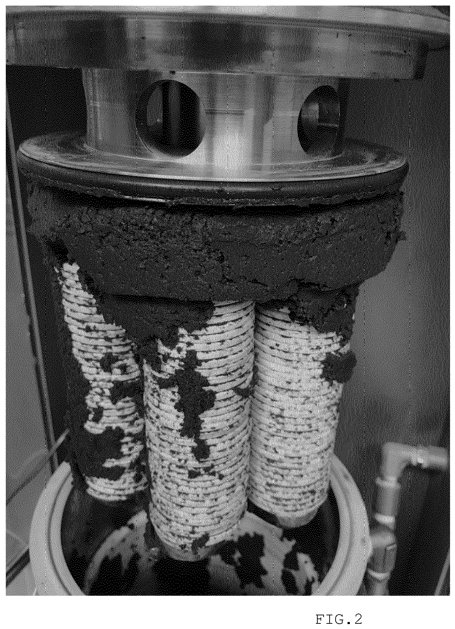

The obtained mixture exemplified on Fig. 2 could be used directly in the cosmetic products or detergent or could be dried and thereafter diluted to the desired consistency.

Essentially the same experiment has been repeated again as seen in Table 2b. In the second experiment the initial temperature was 41°C, which is lower than in the first test and the particle size after 5 minutes of pulverization was larger than in the first experiment.

However, the size of 9.91 µm was reached after 40 minutes and upon temperature increase to 73°C.

Thus, the pulverization was time and temperature dependent and it was possible to pulverise the mixture to the desired size.

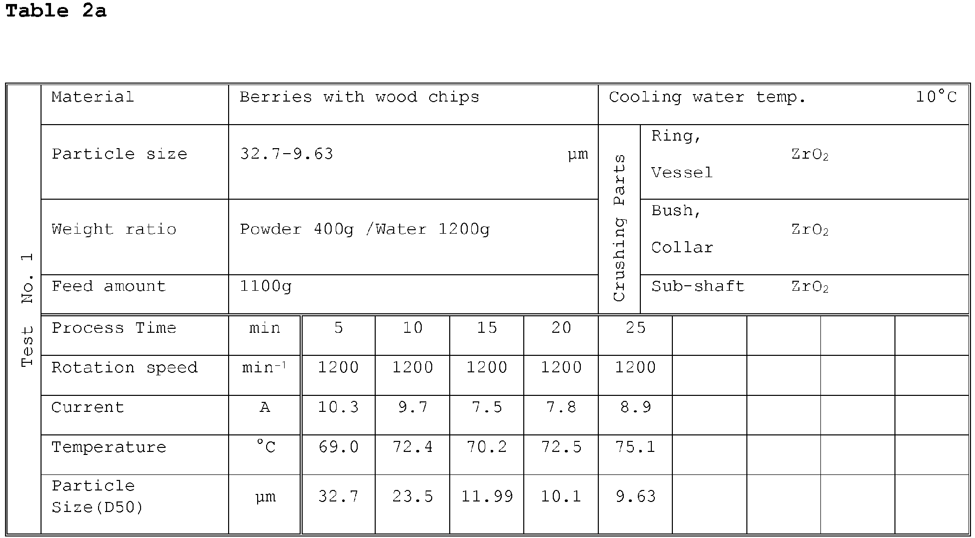

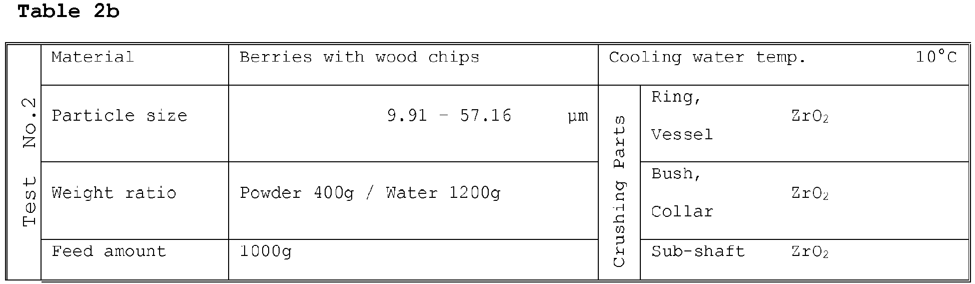

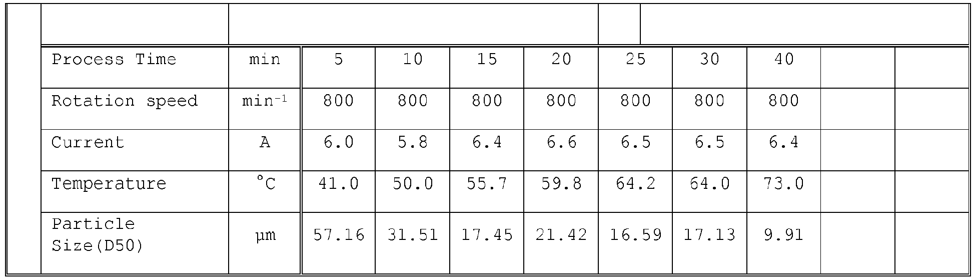

### Example 3.

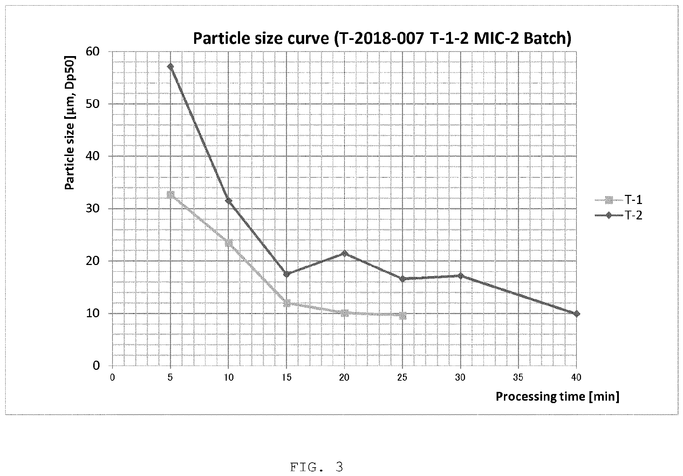

1 part of the mixture as seen on Fig.3, 0.5 coconut oil, pineapple fragrance oil were mixed together at room temperature. The resulting mixture was packaged in a plastic container and used as a body scrub.

## Claims

1. A method for producing wood particles microbeads enriched in berry extract, the method comprising
- providing wood particles and berries, said wood particles having a length in its largest dimension of 3 mm or less,
- mixing wood particles with berries in a ratio between 1:5 to 5:1 of wood to berries (w/w),
- heating said mixture of wood particles and barriers at between 40°C to 75°C for a time sufficient for wood particles to absorb berry extract, thereby forming a first mixture of wood particles enriched with the berry extract, wherein water content of said wood particles enriched with berry extract in the first mixture is 75% or less,
- subjecting said first mixture to size reduction procedure, thereby obtaining a second mixture of wood particles microbeads enriched with berry extract, wherein said microbeads have a size of 1 millimetre or less in their largest dimension and are uniformed in size.

2. The method according to claim 1, wherein said second mixture is subjected to drying at a temperature of between 40°C to 75°C for a time sufficient to decrease water content in said second mixture to 70% or less, preferably 60% or less, preferably 50% or less, more preferably 40% or less, more preferably 30% or less, more preferably 20% or less, more preferably 10 % or less.

3. The method according to any of claims 1 to 3, wherein said second mixture is optionally dried to a water content of 50% or less, 30% or less, 15% or less, 5% or less.

4. The method according to claim 4, wherein said second mixture is a powder.

5. The method according to any of claims 3 or 4, wherein said second mixture is further diluted with diluent to a water content sufficient for a subsequent use.

6. The method according to any of claims 1 to 5, wherein said wood particles are selected from hard wood or soft wood.

7. The method according to claim 6, wherein the soft wood is selected from cedar, juniper, pine redwood, spruce.

8. The method according to claim 6, wherein the hard wood is selected from alder, balsa, beech, birch, hickory, mahogany, maple, oak, teak, and walnut.

9. The method according to any of the preceding claims wherein the berries are selected from bilberries, lingberries, blueberries, raspberries, red currants or black currants.

10. The method according to any of the preceding claims wherein the ratio between berries and wood particles is at between 1 part of berries to 5 parts of wood particles (w/w) to 1 part of berries to 1 part of wood particles (w/w).

11. Wood particles microbeads obtained by the method of any of claims 1 to 10.

12. Wood particles microbeads enriched in berry extract, said wood particles of 1 milometer in the largest dimension or less and are uniform in size and a water content of wood particles microbeads is 75% or less.

13. Wood particles microbeads according to claim 12, wherein said wood particles are selected from hard wood or soft wood.

14. Wood particles microbeads according to claim 13, wherein the soft wood is selected from cedar, juniper, pine redwood, spruce.

15. Wood particle microbeads according to claim 13, wherein the hard wood is selected from alder, balsa, beech, birch, hickory, mahogany, maple, oak, teak, and walnut.

16. Wood particle microbeads according to any of claims 13 to 15, wherein the berries are selected from bilberries, lingberries, blueberries, raspberries, red currants or black currants

17. Use of wood particles microbeads as defined in claims 11-17 as an exfoliating and/or abrasive agent in industrial, home and personal care products.

18. Use of wood particles microbeads as defined in claim 17, wherein industrial products are selected from engine grease and oil remover, paint remover, industrial hand wash.

19. Use of wood particles microbeads according to claim 18, wherein said home and personal care products are selected from detergents, soaps, cosmetic products.
